# EUROPÄISCHE PATENTSCHRIFT

(11) **EP 2 519 272 B1**
(45) Veröffentlichungstag und Bekanntmachung des Hinweises auf die Patenterteilung: **26.02.2014**
(21) Anmeldenummer: 11722995.5
(22) Anmeldetag: 02.05.2011
(51) Int. Cl.: A61M 1/06

(54) **ELEKTRISCHE MUTTERMILCHPUMPE**
ELECTRICAL PUMP FOR BREAST MILK
POMPE ÉLECTRIQUE POUR TIRER DU LAIT MATERNEL

(30) Priorität: 03.05.2010 DE 102010019041
(43) Veröffentlichungstag der Anmeldung: 07.11.2012
(73) Patentinhaber: MAPA GmbH, 27404 Zeven (DE)
(72) Erfinder: JÄGER-WALDAU, Reinhold, 27383 Scheessel (DE)
(74) Vertreter: Hauck Patent- und Rechtsanwälte
(86) Internationale Anmeldenummer: PCT/EP2011/002168
(87) Internationale Veröffentlichungsnummer: WO 2011/137994

(56) Entgegenhaltungen:
- EP-A1- 1 221 319
- EP-A2- 1 586 340
- WO-A1-00/01431
- DE-A1- 19 747 842
- US-A1- 2009 099 511
- US-B1- 6 547 756

## Beschreibung

Die Erfindung bezieht sich auf eine elektrische Muttermilchpumpe.

Muttermilchpumpen dienen dem Abpumpen von Muttermilch. Hierzu weisen sie mindestens eine Saugglocke auf, die auf die Mutterbrust aufgesetzt wird. An die Saugglocke wird ein Unterdruck angelegt, um die Milch aus der Mutterbrust abzuziehen. Dieser Unterdruck wird nachfolgend auch als "Saugunterdruck" bezeichnet. Die Saugglocke ist mit einem Behälter verbunden, der die abgesaugte Muttermilch aufnimmt. Der Unterdruck wird mittels einer Pumpe erzeugt, die manuell oder mittels eines Elektromotors angetrieben ist. Bekannt sind mittels eines Elektromotors angetriebene elektrische Muttermilchpumpen, die eine elektronische Steuerung aufweisen, die bestimmte Pumpsequenzen steuern. In diesen Pumpsequenzen wird die Pumpe während definierter Zeiträume ein- und ausgeschaltet, um den Milchfluss zu stimulieren. Die Höhe des Unterdruckes ist einstellbar.

Aus der EP 1 586 340 A2 ist eine elektrische Milchpumpe bekannt, die mit einem Gyroventil gekoppelt ist. Das Gyroventil umfasst eine Fliehkraftsteuerung, die ein verfedertes Belüftungsventil öffnet, wenn der Elektromotor abgeschaltet wird. Das Belüftungsventil belüftet die Saugglocke, um den Unterdruck in den Pumppausen abzubauen. Wenn der Elektromotor läuft, wird das Belüftungsventil durch Federkraft geschlossen, so dass in den Pumpphasen der Unterdruck in der Saugglocke aufgebaut wird. Während der Pumpsequenz wird die Pumpe mehrfach eingeschaltet und ausgeschaltet. Der Unterdruck in den Pumpphasen ist mit Hilfe eines Einstellrädchens einstellbar, das mit einem Einstellventil verbunden ist, über das die Saugglocke permanent belüftet wird. Je nach Einstellung des Ventils baut sich ein mehr oder weniger großer Unterdruck in der Saugglocke auf.

Bei der bekannten elektrischen Muttermilchpumpe läuft der Elektromotor stets mit maximaler Drehzahl. Infolgedessen hat die Muttermilchpumpe einen hohen Geräuschpegel und einen hohen Stromverbrauch. Ferner wirkt sich dies verkürzend auf die Lebensdauer des Elektromotors aus. Ursächlich für den hohen Stromverbrauch ist auch die Fliehkraftsteuerung, auf die ein Teil der Antriebsleistung entfällt. Ferner beruht der hohe Stromverbrauch darauf, dass der Unterdruck sogleich nach dem Abschalten des Motors durch das Gyroventil abgebaut wird, so dass die Auslaufphase des Motors nicht für den Aufbau des Unterdruckes genutzt wird. Durch die längeren Laufzeiten des Elektromotors wird wiederum seine Lebensdauer verkürzt. Der Verlauf des Unterdruckes am Anfang und am Ende der Pumpphase liegt durch die Konstruktion des Gyroventils fest. Spezielle Unterdruckverläufe, die den Milchfluss besonders stimulieren, sind nicht programmierbar.

Weitere Muttermilchpumpen werden in US 2009/099511 A1, WO 00/01431 A1, EP 1 221 319 A1 offenbart.

Davon ausgehend liegt der Erfindung die Aufgabe zugrunde, eine elektrische Muttermilchpumpe zur Verfügung zu stellen, die einen geringeren Geräuschpegel und Verschleiß sowie einen geringeren Stromverbrauch hat.

Die Aufgabe wird durch eine elektrische Muttermilchpumpe mit den Merkmalen des Anspruches 1 gelöst.

Die erfindungsgemäße elektrische Muttermilchpumpe hat
- einem Elektromotor,
- eine von dem Elektromotor angetriebenen Pumpe,
- mindestens eine Saugglocke,
- eine die Saugglocke mit der Pumpe verbindende Saugleitung,
- einen mit der Saugglocke kommunizierend verbundenen Ablauf für Muttermilch,
- eine mit der Saugglocke kommunizierend verbundene Belüftungsleitung,
- ein elektrisch betätigbares Belüftungsventil in der Belüftungsleitung,
- Mittel zum Ein- und Ausschalten des Elektromotors,
- Mittel zum Einstellen der Drehzahl des Elektromotors,
- eine elektronische Steuerung, die mit den Mitteln zum Ein- und Ausschalten, den Mitteln zum Einstellen der Drehzahl, dem Elektromotor und dem elektrisch betätigbaren Belüftungsventil verbunden ist, und die ausgebildet ist, den Elektromotor in Abhängigkeit einer Betätigung der Mittel zum Ein-und Ausschalten ein- und auszuschalten, den Elektromotor mit einer Drehzahl entsprechend der Einstellung der Mittel zum Einstellen der Drehzahl laufen zu lassen und den Elektromotor kontinuierlich zu betreiben und/oder den Elektromotor in Zyklen zu betreiben, in denen der Elektromotor abwechselnd eingeschaltet und abgeschaltet wird, wobei die elektronische Steuerung beim Abschalten des Elektromotors das Belüftungsventil öffnet und beim Einschalten des Elektromotors schließt, und wobei die elektronische Steuerung das Belüftungsventil mit einer Zeitverzögerung nach dem Ausschalten des Elektromotors oder vor dem Ausschalten des Elektromotors öffnet.

Bei der erfindungsgemäßen elektrischen Muttermilchpumpe wird von der elektronischen Steuerung über die Motordrehzahl der Saugunterdruck eingestellt. Dies hat den Vorteil, dass der Elektromotor im Mittel weniger Strom verbraucht und leiser läuft, als wenn er stets mit maximaler Drehzahl betrieben wird. Die Pumpleistung für den Belüftungsstrom der herkömmlichen Muttermilchpumpe muss von dem Elektromotor nicht aufgebracht werden. Der Verschleiß des Elektromotors ist vermindert, da er im Mittel mit reduzierter Drehzahl arbeitet. Außerdem muss der Elektromotor nicht die Leistung für den Betrieb eines Gyroventiles aufbringen. Diese Einsparung wird zwar mehr oder weniger durch den Stromverbrauch des elektrisch betätigbaren Belüftungsventils kompensiert. Das elektrisch betätigbare Belüftungsventil hat jedoch den Vorteil, dass es von der elektronischen Steuerung beliebig verzögert bezüglich des Elektromotors abgeschaltet werden kann, so dass die Auslaufphase der Pumpe für den Aufbau des Saugunterdruckes genutzt werden kann. Deshalb kommt die Muttermilchpumpe mit kürzeren Einschaltphasen des Elektromotors aus, um einen bestimmten Saugunterdruck zu erzeugen. Auch dies mindert den Stromverbrauch, das Laufgeräusch und reduziert den Verschleiß. Andererseits kann das Belüftungsventil gleichzeitig mit dem Elektromotor oder vor dem Abschalten des Elektromotors geöffnet werden, beispielsweise um den Verlauf des Saugunterdruckes am Ende der Pumpphase zu beeinflussen. Ferner kann das Belüftungsventil zu einem beliebigen Zeitpunkt bezüglich des Einschaltens des Elektromotors geschlossen werden, insbesondere um den Verlauf des Saugunterdruckes am Anfang der Pumpphase zu gestalten. Für die Beeinflussung des Verlaufes des Saugunterdruckes am Anfang und/oder Ende der Pumpphase kann von der elektronischen Steuerung auch die Änderung der Drehzahl des Elektromotors gesteuert werden. Durch die Gestaltung des Verlaufes des Saugunterdruckes kann der Milchfluss in vorteilhafter Weise stimuliert werden.

Vorstehende vorteilhafte Wirkungen kommen zum Tragen, wenn die Muttermilchpumpe kontinuierlich betrieben wird, so dass sie zwischen den Zeitpunkten des Einschaltens und des Ausschaltens fortwährend läuft. Die vorteilhaften Wirkungen der Erfindung kommen besonders zum Tragen, wenn der Elektromotor in Zyklen betrieben wird, in denen er wiederholt eingeschaltet und ausgeschaltet wird. Die Erfindung betrifft sowohl Muttermilchpumpen, die ausschließlich kontinuierlich oder ausschließlich in Zyklen betrieben werden können, als auch Muttermilchpumpen, die sowohl kontinuierlich als auch in Zyklen betrieben werden können.

Es versteht sich, dass die Muttermilchpumpe eine Stromversorgung aufweist, die Elektromotor, Belüftungsventil und elektronische Steuerung speist. Hierbei kann es sich insbesondere um eine Batterie, einen Akkumulator, ein Ladegerät bzw. ein Netzteil handeln. Kombinationen der genannten Stromversorgungen sind möglich.

Gemäß einer Ausgestaltung öffnet die elektronische Steuerung das Belüftungsventil mit einer Zeitverzögerung nach dem Ausschalten des Elektromotors. Die Zeitverzögerung kann beispielsweise 0,5 Sekunden betragen, um die Auslaufphase des Elektromotors für den Aufbau des Saugunterdruckes zu nutzen.

Der Elektromotor ist bevorzugt ein Gleichstrommotor mit Schleifern und Kommutatoren. Derartige Motoren, die für eine Betriebsdauer von einigen 100 Stunden konzipiert sind, stehen in kompakter Bauweise und geeigneter Leistungsstärke zur Verfügung. Mit der Erfindung wird die Lebensdauer der Elektromotoren verlängert, weil sie nicht so hoch bestromt werden, nicht mit so hoher Drehzahl und nicht so lange laufen, wie im Stand der Technik. Hierdurch wird insbesondere der Schleiferabtrag eines Gleichstrommotors mit Schleifern vermindert.

Gemäß einer bevorzugten Ausgestaltung ist das Belüftungsventil ein elektromagnetisches Ventil. Weiterhin bevorzugt ist das elektromagnetische Ventil ein Solenoidventil. Es ist aber auch der Einsatz anderer elektrisch betätigbarer Ventile möglich, beispielsweise ein mittels eines Piezoantriebes betätigbares Ventil.

Gemäß einer weiteren Ausgestaltung umfasst die elektronische Steuerung eine Platine mit einem Mikrorechner, in dem verschiedene Programme zur Steuerung des Elektromotors und des Belüftungsventils gespeichert sind, die durch wahlweise Anbringung von Steckbrücken an verschiedenen Positionen der Platine auswählbar sind. Die Steckbrücken verbinden Leiterbahnen der Platine, die mit dem Mikrorechner verbunden sind. Je nachdem, welche Leiterbahnen verbunden sind, wird der Mikrorechner in verschiedenen Schaltzuständen betrieben, in denen der Mikrorechner verschiedene Programme ausführt. Durch Anbringung der Steckbrücken ist es bei der Produktion möglich, gewünschte Programme der Pumpe auszuwählen. Hierdurch kann eine Familie von Muttermilchpumpen mit verschiedenen Pumpsequenzen zur Verfügung gestellt werden. Hierfür ist lediglich die unterschiedliche Anbringung von Steckbrücken erforderlich. Die Kontaktbrücken können beispielsweise in die Platine eingelötete Drahtbrücken sein.

So ist es beispielsweise möglich, durch eine bestimmte Anbringung von Steckbrücken die Pumpsequenzen der Muttermilchpumpe so festzulegen, dass sie in einer ersten Stufe 60 Pumpzyklen pro Minute, wobei jeder Pumpzyklus eine Pumpphase von 0,5 s und eine Belüftungsphase von 0,5 s Dauer umfasst, und in einer zweiten Stufe 30 Pumpzyklen pro Minute, wobei jeder Pumpzyklus eine Pumpphase von 1 s und eine Belüftungsphase von 1 s Dauer umfasst, ausführt. Durch wiederholte Betätigung der Mittel zum Ein- und Ausschalten kann die Pumpsequenz mit der ersten Stufe eingeleitet, der Übergang von der ersten Stufe zur zweiten Stufe und das Ausschalten der Pumpsequenz gesteuert werden. Ferner ist es durch einfache Anbringung von Steckbrücken möglich, den Mikrorechner so zu schalten, dass die Muttermilchpumpe 30 Pumpzyklen pro Minute, wobei jeder Pumpzyklus eine Pumpphase von 1 s und eine Belüftungsphase von 1 s Dauer umfasst, fortwährend ausführt. Durch betätigen der Mittel zum Ein- und Ausschalten können die Pumpsequenzen gestartet und gestoppt werden.

Gemäß einer weiteren Ausgestaltung unterscheiden sich die verschiedenen Pumpsequenzen durch einen oder mehrere der folgenden Parameter: Dauer der Pumpphasen, Dauer der Belüftungsphasen, Höhe des Saugunterdruckes, Höhe eines Restunterdruckes in Belüftungsphasen, Verlauf des Anstiegs des Saugunterdruckes in den Pumpphasen, Verlauf des Abfalls des Saugunterdruckes in den Belüftungsphasen. Die Beeinflussung des Verlaufs des Saugunterdruckes ist durch Steuerung der Pumpendrehzahl und des Zeitpunkts des Schließens und Öffnens des elektrisch betätigbaren Belüftungsventils möglich.

Gemäß einer bevorzugten Ausgestaltung ist die Pumpe eine Membranpumpe. Mittels einer Membranpumpe können bei kompakter Bauweise die erforderlichen Saugunterdrucke in kurzer Zeit aufgebaut werden. Die Saugunterdrucke liegen bevorzugt im Bereich von 330 bis 100 mbar. In den Pumpphasen können diese Saugunterdrucke mittels einer Membranpumpe in einer Zeit im Sekundenbereich oder darunter aufgebaut werden.

Der Ablauf der Muttermilchpumpe wird bevorzugt in einer Öffnung einer Flasche oder eines anderen Sammelbehälters für Muttermilch angeordnet. Gemäß einer weiteren Ausgestaltung ist zwischen der Saugglocke und dem Ablauf für Muttermilch ein Milchablaufventil vorhanden. Das Milchablaufventil öffnet, wenn sich Milch darüber ansammelt, um die Muttermilch z.B. in eine Flasche abzulassen.

Gemäß einer weiteren Ausgestaltung ist das Milchablaufventil ein Entenschnabelventil. Das Entenschnabelventil ist ein im Querschnitt V-förmiges Ventil mit einem Schlitz an der Kontaktlinie zwischen den Schenkeln des V. Das Milchablassventil öffnet, wenn das Entenschnabelventil zwischen den Schenkeln des V hinreichend mit Muttermilch gefüllt ist, um diese z.B. in eine Flasche abzulassen.

Gemäß einer Ausgestaltung ist zwischen der Saugglocke und der Pumpe ein Schwimmerventil angeordnet. Das Schwimmerventil schließt nur dann durch Aufschwimmen gegen einen Ventilsitz der Muttermilchpumpe, wenn die Milch nicht schnell genug über das Milchablaufventil in den Sammelbehälter/Flasche abgeführt werden kann. Dies ist der Fall, wenn der Raum zwischen Milchablaufventil und Saugglocke gefüllt ist. Das Schwimmerventil verhindert bei "vollständiger Abdichtung", dass Flüssigkeit in die Pumpe gesogen wird. Ausgetrocknete Milch kann die Einlass- und Auslassventile der Pumpe verkleben und blockieren.

Gemäß einer weiteren Ausgestaltung umfasst die Muttermilchpumpe einen Verbindungskanal, an dem unten das Milchablaufventil und oben das Schwimmerventil angeordnet ist, wobei zwischen Milchablaufventil und Schwimmerventil die Saugglocke angeschlossen ist und sich oben an das Schwimmerventil der Saugleitung anschließt. Bevorzugt ist der Verbindungskanal mit einem Deckel zum Aufsetzen auf eine Flasche verbunden. Bei diesem Deckel handelt es sich bevorzugt um einen Schraubdeckel.

Weiterhin bevorzugt weist der Deckel einen Belüftungskanal zum Belüften einer Flasche auf, an deren Flaschenöffnung der Deckel fixiert ist.

Gemäß einer weiteren Ausgestaltung sind der Elektromotor, die Pumpe, die elektronische Steuerung, das Belüftungsventil und eine Stromversorgung in einem Gehäuse zusammengefasst.

Gemäß einer weiteren Ausgestaltung ist das Gehäuse mit dem Verbindungskanal starr verbunden bzw. der Verbindungskanal zumindest teilweise in das Gehäuse integriert.

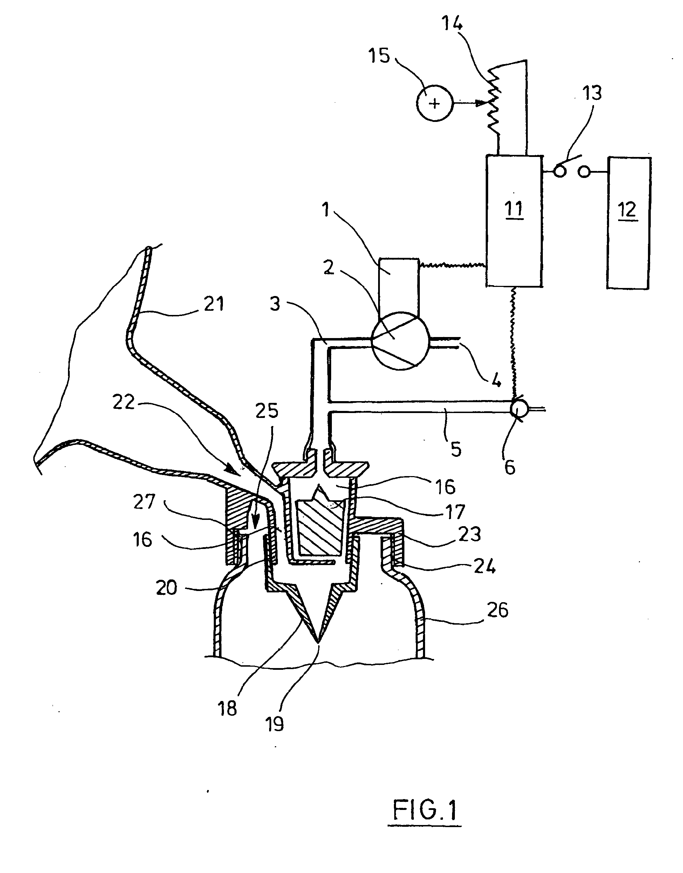

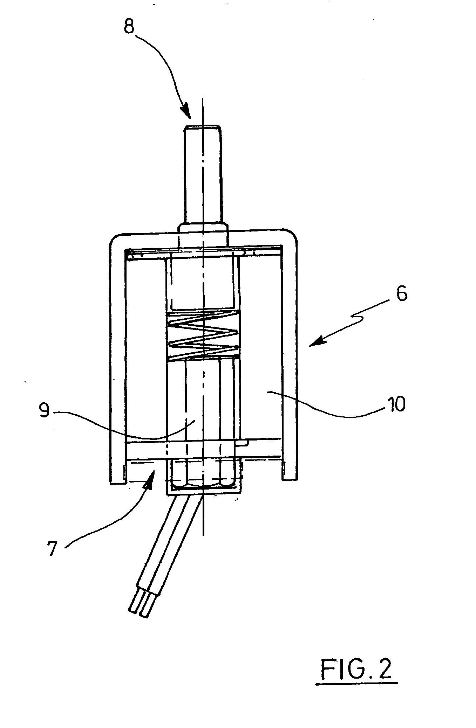

Die Erfindung wird nachfolgend anhand der anliegenden Zeichnungen erläutert, die in Fig. 1 eine erfindungsgemäße Muttermilchpumpe in einem grobschematischen Vertikalschnitt und in Fig. 2 ein Belüftungsventil in einem Vertikalschnitt zeigen.

Gemäß Fig. 1 weist die Muttermilchpumpe einen als Gleichstrommotor mit Schleifen und Konstator ausgebildeten Elektromotor 1 und eine damit mechanisch gekoppelte Pumpe 2 auf, die als Membranpumpe ausgebildet ist. An den Eingang der Pumpe 2 ist eine Saugleitung 3 angeschlossen. Der Ausgang der Pumpe 2 ist mit einem Luftauslass 4 verbunden.

Die Saugleitung 3 ist mit einer Belüftungsleitung 5 verbunden, in der ein elektrisch betätigbares Belüftungsventil 6 angeordnet ist, das zur Atmosphäre hin lüftet. Das Belüftungsventil 6 ist gemäß Fig. 2 als Solenoidventil mit Einlass 7, Auslass 8, beweglichem ferromagnetischem Ventilkörper 9 und Elektromagnet 10 ausgeführt.

Gemäß Fig. 1 umfasst die Muttermilchpumpe eine elektronische Steuerung 11 und eine Stromversorgung 12. Die elektronische Steuerung 11 ist mit einem Ein-Ausschalter 13 und Mittel zum Einstellen 14 der Drehzahl des Elektromotors 1 verbunden, die ein Einstellrädchen 15 aufweisen.

Die Saugleitung 3 ist mit dem oberen Ende eines Verbindungskanals 16 verbunden. Im oberen Ende des Verbindungskanals 16 ist ein Schwimmerventil 17 angeordnet

Ferner ist der Verbindungskanal 16 am unteren Ende mit einem Milchablassventil 18 in Form eines Entenschnabelventils mit einem Ventilschlitz 19 am unteren Ende bestückt. Dieses ist auf ein stutzenförmiges Rohrstück 20 am unteren Ende des Verbindungskanals 16 aufgeschoben oder in dieses eingesteckt.

Ferner ist der Verbindungskanal 16 seitlich starr mit einer Saugglocke 21 verbunden. Die Saugglocke 21 erweitert sich nach außen und ist an ihrem inneren Ende über eine Durchgangsöffnung 22 mit dem Verbindungskanal 16 verbunden.

Der Verbindungskanal 16 ist außen mit einem Schraubring 23 verbunden, der auf ein Schraubgewinde 24 an der Flaschenöffnung 25 einer Flasche 26 aufschraubbar ist, so dass das Milchablaufventil 18 in die Flaschenöffnung 25 hineinsteht. Der Schraubring 23 weist einen integrierten Luftkanal 27 auf.

Durch Betätigen des Ein-/Ausschalters 13 ist die Muttermilchpumpe einschaltbar. Die elektronische Steuerung 11 steuert die Drehzahl der Pumpe 2 entsprechend der mittels der Mittel zum Einstellen 14 eingestellten Pumpendrehzahl. Die elektronische Steuerung 11 steuert den Elektromotor 1 so, dass die Pumpe 2 während einer bestimmten Anzahl Pumpzyklen in einer Zeitspanne eingeschaltet und wieder abgeschaltet wird. In den Pumpphasen schaltet die elektronische Steuerung 11 den Elektromotor 1 an und schließt das elektrisch betätigbare Belüftungsventil 6. Beim Abschalten der Pumpe 2 in den Belüftungsphasen öffnet die elektronische Steuerung 11 das elektrisch betätigbare Belüftungsventil 6.

In den Pumpphasen wird über die Saugleitung 3 und das Schwimmerventil 17 im Verbindungskanal 16 ein Unterdruck aufgebaut. Infolgedessen wird durch die Saugglocke 21 Milch aus einer Mutterbrust abgesaugt. Diese sammelt sich auf dem Milchablassventil 18 und läuft in die Flasche 26 ab, wenn eine hinreichende Menge angesaugt ist, um den Ventilschlitz 19 am unteren Ende des Milchablassventils 18 zu öffnen.

Nach einer vorgegebenen Anzahl Pumpzyklen oder nach Abschalten der Mittel zum Ein- und Ausschalten 13 wird die Pumpe 2 abgeschaltet.

## Patentansprüche

1. Elektrische Muttermilchpumpe mit
- einem Elektromotor (1),
- einer von dem Elektromotor (1) angetriebenen Pumpe (2),
- mindestens einer Saugglocke (21),
- einer die Saugglocke (21) mit der Pumpe (2) verbindenden Saugleitung (3),
- einem mit der Saugglocke (21) kommunizierend verbundenen Ablauf für Muttermilch,
- einer mit der Saugglocke (21) kommunizierend verbundenen Belüftungsleitung (5),
- einem elektrisch betätigbaren Belüftungsventil (6) in der Belüftungsleitung (5),
- Mitteln zum Ein- und Ausschalten (13) des Elektromotors (1),
- Mitteln zum Einstellen (14) der Drehzahl des Elektromotors (1),
- einer elektronischen Steuerung (11), die mit den Mitteln zum Ein- und Ausschalten (13), den Mitteln zum Einstellen (14) der Drehzahl, dem Elektromotor (1) und dem elektrisch betätigbaren Belüftungsventil (6) verbunden ist und die ausgebildet ist, den Elektromotor (1) in Abhängigkeit von einer Betätigung der Mittel zum Ein- und Ausschalten (13) einund auszuschalten, den Elektromotor (1) mit einer Drehzahl entsprechend der Einstellung der Mittel zum Einstellen (14) der Drehzahl laufen zu lassen und **dadurch gekennzeichnet, dass** die elektronische Steuerung weiter ausgebildet ist, den Elektromotor (1) in Zyklen zu betreiben, in denen der Elektromotor (1) abwechselnd eingeschaltet und abgeschaltet wird, wobei die elektronische Steuerung (11) beim Abschalten des Elektromotors (1) das Belüftungsventil (6) öffnet und beim Einschalten des Elektromotors (1) schließt.
wobei die elektronische Steuerung (11) das Belüftungsventil (6) mit einer Zeitverzögerung nach dem Ausschalten des Elektromotors (1) oder vor dem Ausschalten des Elektromotors (1) öffnet.

2. Muttermilchpumpe nach Anspruch 1, bei der das Belüftungsventil (6) ein elektromagnetisches Ventil ist.

3. Muttermilchpumpe nach Anspruch 2, bei der das elektromagnetische Ventil (6) ein Solenoidventil ist.

4. Muttermilchpumpe nach einem der Ansprüche 1 bis 3, bei der die elektronische Steuerung (11) eine Platine mit einem Mikrorechner umfasst, in dem verschiedene Programme gespeichert sind, die durch wahlweise Anbringung von Steckbrücken an verschiedenen Positionen der Platine auswählbar sind.

5. Muttermilchpumpe nach Anspruch 4, bei der die verschiedenen Programme einer elektronischen Steuerung (11) verschiedene Pumpsequenzen steuern.

6. Muttermilchpumpe nach Anspruch 4 oder 5, bei der die verschiedenen Fumpzyklen sich durch einen oder mehrere der folgenden Parameter unterscheiden: Dauer der Pumpphasen, Dauer der Belüftungsphasen, Höhe des Saugunterdruckes, Höhe eines Restunterdruckes in Belüftungsphasen, Verlauf des Anstiegs des Saugunterdruckes in den Pumpphasen, Verlauf des Abfalls des Saugunterdruckes in den Belüftungsphasen.

7. Muttermilchpumpe nach einem der Ansprüche 1 bis 6, bei der die Pumpe (2) eine Membranpumpe ist.

8. Muttermilchpumpe gemäß einem der Ansprüche 1 bis 7, bei der zwischen der Saugglocke (21) und dem Ablauf für Muttermilch ein Milchablaufventil (18) vorhanden ist, das öffnet, wenn Milch sich darüber ansammelt.

9. Muttermilchpumpe gemäß Anspruch 8, bei der das Milchablaufventil (18) ein Entenschnabelventil ist.

10. Muttermilchpumpe nach Anspruch 8 oder 9, bei der zwischen Saugglocke (21) und Pumpe (2) ein Schwimmerventil (17) angeordnet ist, das schließt, wenn Milch oberhalb des Milehablaufventils (18) bis zum Schwimmerventil (17) ansteigt.

11. Muttermilchpumpe gemäß einem der Ansprüche 1 bis 10, die einen Verbindungskanal (16) aufweist, an dem unten das Milchablaufventil (18) und oben das Schwimmerventil (17) angeordnet ist, wobei zwischen Milchablaufventil (18) und Schwimmerventil (17) die Saugglocke (21) angeschlossen ist und sich oben an das Schwimmerventil (17) die Saugleitung (3) anschließt.

12. Muttermilchpumpe nach Anspruch 11, bei der der Verbindungskanal (16) außen mit einem Deckel (23) zum Aufsetzen auf eine Flasche (26) verbunden ist.

13. Muttermilchpumpe nach Anspruch 12, bei der der Deckel (23) einen Belüftungskanal (27) zum Belüften einer Flasche (26) aufweist.

14. Muttermilchpumpe gemäß einem der Ansprüche 1 bis 13, bei der der Elektromotor (1), die Pumpe (2), die elektronische Steuerung (11), das Belüftungsventil (6), eine elektronische Stromversorgung (11) und der Verbindungskanal (16) zumindest teilweise in ein Gehäuse integriert sind.

## Claims

1. An electrical pump for breast milk comprising
- an electric motor (1),
- a pump (2) driven by the electric motor (1),
- at least one suction cup (21),
- a suction line (3) connecting the suction cup (21) to the pump (2),
- an outlet for breast milk connected so as to communicate with the suction cup (21),
- a ventilation line (5) connected so as to communicate with the suction cup (21),
- an electrically actuatable ventilation valve (6) in the ventilation line (5),
- means for switching on and off (13) the electric motor (1),
- means for adjusting (14) the rotational speed of the electric motor (1),
- an electronic control unit (11) which is connected to the means for switching on and off (13), the means for adjusting (14) the rotational speed, the electric motor (1) and the electrically actuatable ventilation valve (6) and which is configured to switch the electric motor (1) on and off, depending on an actuation of the means for switching on and off (13), to permit the electric motor (1) to run at a rotational speed according to the adjustment of the means for adjusting (14) the rotational speed, **characterized in that** the electronic control unit is further configured to operate the electric motor (1) in cycles, in which the electric motor (1) is alternately switched on and switched off, the electronic control unit (11) opening the ventilation valve (6) when the electric motor (1) is switched off and closing said ventilation valve when the electric motor (1) is switched on, the electronic control unit (11) opening the ventilation valve (6) with a time delay after the electric motor (1) is switched off or before the electric motor (1) is switched off.

2. The pump for breast milk according to Claim 1, in which the ventilation valve (6) is an electromagnetic valve.

3. The pump for breast milk according to Claim 2, in which the electromagnetic valve (6) is a solenoid valve.

4. The pump for breast milk according to one of Claims 1 to 3, in which the electronic control unit (11) comprises a printed circuit board with a microcomputer in which different programs are stored, said programs being able to be selected by optionally attaching jumpers at different positions of the printed circuit board.

5. The pump for breast milk according to Claim 4, in which the different programs of an electronic control unit (11) control different pumping sequences.

6. The pump for breast milk according to Claim 4 or 5, in which the different pumping cycles differ by one or more of the following parameters: duration of pumping phases, duration of ventilation phases, level of negative suction pressure, level of residual negative pressure in the ventilation phases, course of the rise in negative suction pressure in the pumping phases, course of the fall in negative suction pressure in the ventilation phases.

7. The pump for breast milk according to one of Claims 1 to 6, in which the pump (2) is a membrane pump.

8. The pump for breast milk according to one of Claims 1 to 7, in which a milk outlet valve (18) is present between the suction cup (21) and the outlet for breast milk, said milk outlet valve opening when milk collects above said milk outlet valve.

9. The pump for breast milk according to Claim 8, in which the milk outlet valve (18) is a duck-bill valve.

10. The pump for breast milk according to Claim 8 or 9, in which a floating valve (17) is arranged between the suction cup (21) and the pump (2), said floating valve closing when milk above the milk outlet valve (18) rises up to the floating valve (17).

11. The pump for breast milk according to one of Claims 1 to 10, which comprises a connecting channel (16) on which the milk outlet valve (18) is arranged at the bottom and the floating valve (17) is arranged at the top, the suction cup (21) being attached between the milk outlet valve (18) and the floating valve (17), and the suction line (3) being connected to the floating valve (17) at the top.

12. The pump for breast milk according to Claim 11, in which the connecting channel (16) is connected externally to a lid (23) for positioning onto a bottle (26).

13. The pump for breast milk according to Claim 12, in which the lid (23) has a ventilation channel (27) for ventilating a bottle (26).

14. The pump for breast milk according to one of Claims 1 to 13, in which the electric motor (1), the pump (2), the electronic control unit (11), the ventilation valve (6), an electronic power supply (11) and the connecting channel (16) are at least partially integrated in a housing.

## Revendications

1. Pompe électrique pour tirer du lait maternel, avec
- un moteur électrique (1),
- une pompe (2) entraînée par le moteur électrique (1),
- au moins une cloche d'aspiration (21),
- une conduite d'aspiration (3) reliant la cloche d'aspiration (21) à la pompe (2),
- une évacuation pour le lait maternel, reliée de façon communicante à la cloche d'aspiration (21),
- une conduite d'aération (5) reliée de façon communicante à la cloche d'aspiration (21),
- une soupape d'aération (6) actionnable de façon électrique, située dans la conduite d'aération (5),
- des moyens pour la mise en marche et l'arrêt (13) du moteur électrique (1),
- des moyens pour le réglage (14) de la vitesse de rotation du moteur électrique (1),
- une commande électronique (11) reliée aux moyens pour la mise en marche et l'arrêt (13), les moyens pour le réglage (14) de la vitesse de rotation, le moteur électrique (1) et la soupape d'aération (6) actionnable de façon électrique, tout en étant conçue pour mettre en marche et arrêter le moteur électrique (1) en fonction d'un actionnement des moyens pour la mise en marche et l'arrêt (13), pour faire tourner le moteur électrique (1) à une vitesse de rotation correspondant au réglage des moyens pour le réglage (14) de la vitesse de rotation, **caractérisée en ce que** la commande électronique est en outre conçue pour faire fonctionner le moteur électrique (1) par cycles, au cours desquels le moteur électrique (1) est mis en marche et arrêté en alternance, dans laquelle la commande électronique (11) ouvre la soupape d'aération (6) lors de l'arrêt du moteur électrique (1) et la ferme lors de la mise en marche du moteur électrique (1), dans laquelle la commande électronique (11) ouvre la soupape d'aération (6) avec un décalage dans le temps après l'arrêt du moteur électrique (1) ou avant l'arrêt du moteur électrique (1).

2. Pompe électrique pour tirer du lait maternel selon la revendication 1, dans laquelle la soupape d'aération (6) est une soupape électromagnétique.

3. Pompe électrique pour tirer du lait maternel selon la revendication 2, dans laquelle la soupape électromagnétique (6) est une soupape à solénoïde.

4. Pompe électrique pour tirer du lait maternel selon l'une des revendications 1 à 3, dans laquelle la commande électronique (11) comprend une platine avec un micro-ordinateur, dans lequel sont stockés différents programmes, lesquels peuvent être sélectionnés en fixant des cavaliers au choix à différents endroits de la platine.

5. Pompe électrique pour tirer du lait maternel selon la revendication 4, dans laquelle les différents programmes d'une commande électronique (11) commandent différentes séquences de pompage.

6. Pompe électrique pour tirer du lait maternel selon la revendication 4 ou 5, dans laquelle les différents cycles de pompage se distinguent par un ou plusieurs des paramètres suivants : durée des phases de pompage, durée des phases d'aération, niveau de la dépression d'aspiration, niveau d'une dépression résiduelle dans les phases d'aération, évolution de l'augmentation d'une dépression d'aspiration dans les phases de pompage, évolution de la chute de la dépression d'aspiration dans les phases d'aération.

7. Pompe électrique pour tirer du lait maternel selon l'une des revendications 1 à 6, dans laquelle la pompe (2) est une pompe à membrane.

8. Pompe électrique pour tirer du lait maternel selon l'une des revendications 1 à 7, dans laquelle une soupape d'évacuation de lait (18) est prévue entre la cloche d'aspiration (21) et l'évacuation pour le lait maternel, laquelle s'ouvre lorsque du lait s'accumule au-dessus.

9. Pompe électrique pour tirer du lait maternel selon la revendication 8, dans laquelle la soupape d'évacuation de lait (18) est une soupape en bec de canard.

10. Pompe électrique pour tirer du lait maternel selon la revendication 8 ou 9, dans laquelle une soupape à flotteur (17) est agencée entre la cloche d'aspiration (21) et la pompe (2), laquelle se ferme lorsque le lait monte au-dessus de la soupape d'évacuation de lait (18) jusqu'à la soupape à flotteur (17).

11. Pompe électrique pour tirer du lait maternel selon l'une des revendications 1 à 10, comportant un canal de liaison (16) en haut duquel est agencée la soupape d'évacuation de lait (18) et en bas duquel est agencée la soupape à flotteur (17), dans laquelle la cloche d'aspiration (21) est raccordée entre la soupape d'évacuation de lait (18) et la soupape à flotteur (17), et la conduite d'aspiration (3) est raccordée en haut à la soupape à flotteur (17).

12. Pompe électrique pour tirer du lait maternel selon la revendication 11, dans laquelle le canal de liaison (16) est relié extérieurement à un couvercle (23) à placer sur une bouteille (26).

13. Pompe électrique pour tirer du lait maternel selon la revendication 12, dans laquelle le couvercle (23) comporte un canal d'aération (27) pour l'aération d'une bouteille (26).

14. Pompe électrique pour tirer du lait maternel selon l'une des revendications 1 à 13, dans laquelle le moteur électrique (1), la pompe (2), la commande électronique (11), la soupape d'aération (6), une alimentation électrique électronique (11) et le canal de liaison (16) sont au moins partiellement intégrés dans un boîtier.
